# DEMANDE DE BREVET EUROPEEN

(11) **EP 4 809 917 A1**
(43) Date de publication de la demande: **23.09.2026**
(21) Numéro de dépôt: 26164162.5
(22) Date de dépôt: 12.03.2026
(51) Int. Cl.: A61B 5/107

(54) **PROCEDE ET SYSTEME D'ACQUISITION D'UN SIGNAL REPRESENTATIF D'UN DIAMETRE D'UN VAISSEAU SANGUIN**

(30) Priorité: 20.03.2025 FR 2502838
(71) Demandeur: Commissariat à l'Energie Atomique et aux Energies Alternatives, 75015 Paris (FR)
(72) Inventeur: VERNAY, Augustin, 38054 GRENOBLE CEDEX 09 (FR); BLANQUER, Guillaume, 38054 GRENOBLE CEDEX 09 (FR); PERRIOLLAT, Mathieu, 38054 GRENOBLE CEDEX 09 (FR); BLANDIN, Pierre, 38054 GRENOBLE CEDEX 9 (FR); GERBELOT BARILLON, Rémi, 38054 GRENOBLE CEDEX 09 (FR)
(74) Mandataire: Cabinet Beaumont

(57) **Abrégé**

La présente description concerne un procédé d'acquisition d'un signal, comprenant une phase de calibration comprenant :
1) déterminer un signal de référence représentatif de l'évolution du diamètre du vaisseau sanguin, au moyen d'un capteur ultrasonore (220) ;
2) acquérir un signal optique, au moyen d'un capteur photopléthysmographique (100) ;
3) déterminer, à partir du signal optique et du signal de référence, un modèle mathématique adapté à générer, à partir du seul signal optique, un signal estimé représentatif de l'évolution du diamètre du vaisseau sanguin pendant la phase de calibration,
le procédé comprenant en outre, après la phase de calibration :
4) mesurer un signal optique représentatif de l'évolution du vaisseau sanguin, au moyen du capteur photopléthysmographique (100) ; et
5) calculer un signal estimé représentatif de l'évolution du diamètre du vaisseau sanguin, à partir du signal mesuré à l'étape 4) et du modèle mathématique.

## Description

### Domaine technique

La présente description concerne de façon générale des mesures de paramètres cardio-vasculaires et en particulier un procédé et un système d'acquisition d'un signal représentatif de l'évolution d'un diamètre d'un vaisseau sanguin au moyen d'un capteur optique.

### Technique antérieure

Une mesure d'une évolution d'un diamètre ou d'une grandeur caractéristique d'un diamètre d'un vaisseau sanguin, d'une artère par exemple, au cours du temps permet un suivi de paramètres cardio-vasculaires tels que la pression artérielle, le rythme cardiaque, la saturation pulsée de l'hémoglobine en oxygène (SpO2), la rigidité artérielle, le pouls, etc. Ces paramètres peuvent être utilisés pour un suivi médical, un suivi de performances physiques et sportives, etc.

La pression artérielle est communément mesurée par un tensiomètre comprenant un brassard gonflable. Cependant un tel brassard est encombrant pour l'utilisateur et peut perturber la mesure, par exemple lors d'une utilisation continue, notamment au cours d'une nuit.

Il est connu de mesurer le diamètre d'un vaisseau sanguin à partir d'images obtenues au moyen d'un capteur ultrasonore. Cependant, l'imagerie ultrasonore présente des inconvénients. En particulier, elle nécessite une chaine d'acquisition complexe et une puissance de calcul importante qui rendent la solution difficilement miniaturisable. L'imagerie ultrasonore implique en outre une consommation électrique relativement élevée, la génération d'un volume de données important, et la nécessité d'utiliser un matériau de couplage acoustique pour l'obtention d'images de qualité suffisante.

Il est par ailleurs connu de mesurer une fréquence cardiaque ou un pouls au moyen d'un capteur optique aussi appelé capteur photopléthysmographique, pouvant être intégré à un dispositif portatif, par exemple une montre portée au poignet d'un utilisateur. De tels capteurs sont relativement peu encombrants et nécessitent des ressources énergétiques et calculatoires limitées. Ils ne permettent cependant pas de mesurer de façon fiable et précise l'évolution au cours du temps d'un diamètre ou d'une grandeur caractéristique d'un diamètre d'un vaisseau sanguin.

Il existe un besoin de palier au moins en partie certains des inconvénients des solutions connues.

### Résumé de l'invention

Un mode de réalisation prévoit un procédé d'acquisition d'un signal représentatif d'un diamètre d'un vaisseau sanguin, comprenant une phase de calibration, la phase de calibration comprenant les étapes suivantes :
1) déterminer un signal de référence représentatif de l'évolution du diamètre du vaisseau sanguin pendant une période de calibration, au moyen d'un capteur ultrasonore ;
2) acquérir un signal optique représentatif de l'évolution du vaisseau sanguin pendant la période de calibration, au moyen d'un capteur photopléthysmographique ;
3) déterminer, à partir du signal optique mesuré à l'étape 2) et du signal de référence déterminé à l'étape 1), un modèle mathématique adapté à générer, à partir du seul signal optique mesuré à l'étape 2), un signal estimé représentatif de l'évolution du diamètre du vaisseau sanguin pendant la période de calibration,
le procédé comprenant en outre, après la phase de calibration, les étapes suivantes :
4) mesurer un signal optique représentatif de l'évolution du vaisseau sanguin pendant une période d'acquisition, au moyen du capteur photopléthysmographique ; et
5) calculer un signal estimé représentatif de l'évolution du diamètre du vaisseau sanguin pendant la période d'acquisition, à partir du signal mesuré à l'étape 4) et du modèle mathématique déterminé à l'étape 3).

Selon un mode de réalisation, l'étape 1) comprend l'acquisition d'un ou plusieurs signaux ultrasonores et la détermination du signal de référence à partir desdits signaux ultrasonores.

Selon un mode de réalisation, le signal optique acquis à l'étape 2) comprend une pluralité de signaux optiques acquis respectivement dans plusieurs configurations d'acquisitions.

Selon un mode de réalisation, le capteur photopléthysmographique comprend au moins un émetteur et au moins un récepteur et dans lequel une configuration d'acquisition comprend une longueur d'onde d'émission par l'émetteur et une distance entre l'émetteur et le récepteur.

Selon un mode de réalisation, à l'étape 3), le modèle mathématique est de la forme :
$Y \left(t\right) = f \left(\left\{S\left(t\right)\right\}, p\right)$
où Y désigne le signal estimé, t désigne une variable temporelle, f désigne une fonction déterminée, S désigne le signal optique acquis à l'étape 2) et p désigne un vecteur de paramètre comprenant un ou plusieurs paramètres à déterminer.

Selon un mode de réalisation, la fonction f est une fonction linéaire, le vecteur de paramètre p de la fonction linéaire étant déterminé par régression linéaire.

Selon un mode de réalisation, ladite régression linéaire est une régression linéaire régularisée, par exemple une régression Lasso.

Selon un mode de réalisation, la fonction f est définie comme suit :
$f \left(\left\{S\left(t\right)\right\}, p\right) = A ∗ ln \left(S\left(t\right)/{S}_{0}\right) + B$
où S₀ désigne l'intensité d'une onde émise par le capteur photopléthysmographique à l'étape 2) et A et B sont deux valeurs de paramètres du vecteur de paramètre p.

Selon un mode de réalisation:
$A = \frac{\left(y1-y2\right)}{ln \left(\frac{s 1}{s 2}\right)}$
et
$B = {y}_{1} \frac{{y}_{1} - {y}_{2}}{ln \left(\frac{{s}_{1}}{{s}_{2}}\right)} ln \left(\frac{{s}_{1}}{{s}_{0}}\right)$
où y1 et y2 désignent deux valeurs de la grandeur représentative du diamètre du vaisseau sanguin déterminée à l'étape 3) et s1 et s2 désignent deux valeurs du signal optique mesuré à l'étape 2), y1 étant obtenue à partir d'une première mesure synchrone avec s1 et y2 étant obtenue à partir d'une deuxième mesure synchrone avec s2.

Selon un mode de réalisation, la fonction f est définie par simulation numérique.

Selon un mode de réalisation, un apprentissage supervisé est utilisé pour déterminer la fonction f.

Selon un mode de réalisation, le vaisseau sanguin est une artère.

Un mode de réalisation prévoit un système d'acquisition d'un signal représentatif d'un diamètre d'un vaisseau sanguin comprenant :
- un capteur ultrasonore ;
- un capteur photopléthysmographique ; et
- un dispositif de traitement de données configuré pour, lors d'une phase de calibration :
   1) déterminer un signal de référence représentatif de l'évolution du diamètre du vaisseau sanguin pendant une période de calibration, au moyen du capteur ultrasonore ;
   2) acquérir un signal optique représentatif de l'évolution du vaisseau sanguin pendant la période de calibration, au moyen du capteur photopléthysmographique ;
   3) déterminer, à partir du signal optique mesuré à l'étape 2) et du signal de référence déterminé à l'étape 1), un modèle mathématique adapté à générer, à partir du seul signal optique mesuré à l'étape 2), un signal estimé représentatif de l'évolution du diamètre du vaisseau sanguin pendant la période de calibration,
   le procédé comprenant en outre, après la phase de calibration, les étapes suivantes :
   4) mesurer un signal optique représentatif de l'évolution du vaisseau sanguin pendant une période d'acquisition, au moyen du capteur photopléthysmographique ; et
   5) calculer un signal estimé représentatif de l'évolution du diamètre du vaisseau sanguin pendant la période d'acquisition, à partir du signal mesuré à l'étape 4) et du modèle mathématique déterminé à l'étape 3).

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :
la figure 1A et la figure 1B représentent de façon schématique, une acquisition d'un signal par un capteur optique selon un mode de réalisation ;
la figure 1C représente de façon schématique un exemple d'évolution d'un signal de photopléthysmographie selon un mode de réalisation ; et
la figure 2 représente de façon schématique, sous forme de blocs, un exemple d'un système d'acquisition d'un signal représentatif d'un diamètre d'un vaisseau sanguin selon un mode de réalisation ;
la figure 3 représente de façon schématique, sous forme de blocs, un exemple d'un procédé d'acquisition d'un signal représentatif d'un diamètre d'un vaisseau sanguin selon un mode de réalisation.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Par souci de clarté, seuls les étapes et éléments utiles à la compréhension des modes de réalisation décrits ont été représentés et sont détaillés. En particulier, l'acquisition de signaux représentatifs de l'évolution d'un vaisseau sanguin au moyen d'un capteur ultrasonore ou à partir d'un capteur optique est connue de la personne du métier. Ainsi, ces acquisitions ne seront pas détaillées dans la présente description. De même, la réalisation des capteurs ultrasonores ou optiques ne sera pas détaillée, les modes de réalisation décrits étant compatibles avec les capteurs ultrasonores ou optiques connus, ou la réalisation de ces capteurs étant à la portée de la personne du métier à partir des indications de la présente description. En outre, la détermination d'une grandeur caractéristique du diamètre d'un vaisseau sanguin à partir d'un ou plusieurs signaux ultrasonores est également connue de la personne du métier et ne sera pas détaillée ci-après. De plus, la réalisation des unités ou circuits électroniques de contrôle et de traitement des systèmes décrits n'a pas été détaillée, celle-ci étant à la portée de la personne du métier à partir des indications fonctionnelles de la présente description.

Sauf précision contraire, lorsque l'on fait référence à deux éléments connectés entre eux, cela signifie directement connectés sans éléments intermédiaires autres que des conducteurs, et lorsque l'on fait référence à deux éléments reliés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", etc., ou à des qualificatifs d'orientation, tels que les termes "horizontal", "vertical", etc., il est fait référence sauf précision contraire à l'orientation des figures dans une position normale d'utilisation.

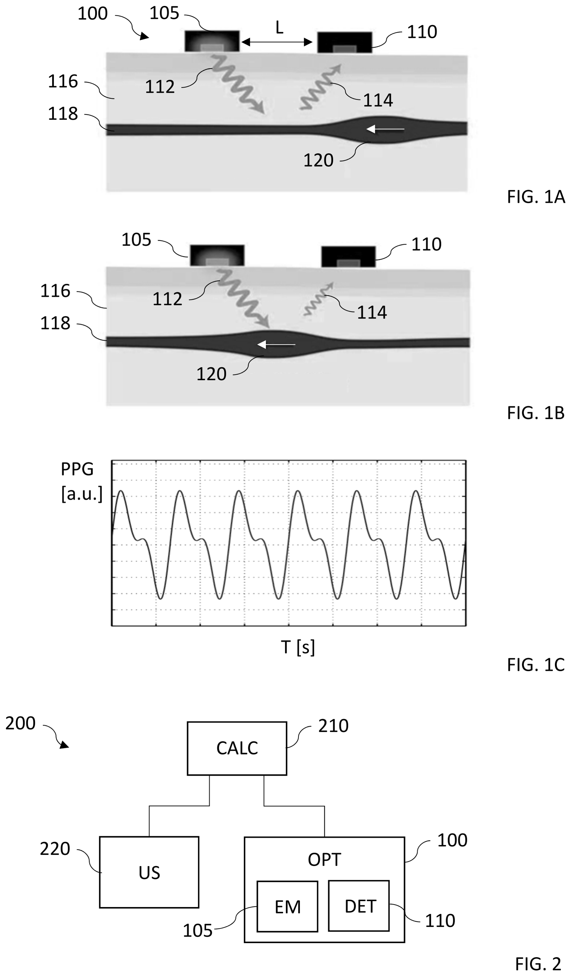

La figure 1A et la figure 1B représentent de façon schématique, une acquisition d'un signal par un capteur optique 100 selon un mode de réalisation.

Le capteur optique 100 est par exemple un capteur de photopléthysmographie.

Dans l'exemple des figures 1A et 1B, le capteur optique 100 comprend un émetteur 105 et un récepteur 110. L'émetteur 105, par exemple une diode, est configuré pour émettre une onde optique incidente 112, par exemple une onde ayant une longueur d'onde dans le spectre visible ou infrarouge ou proche infrarouge. Le récepteur 110, par exemple une photodiode, est configuré pour détecter une partie de l'onde optique incidente 112 qui est réfléchie ou rétrodiffusée 114.

A titre d'exemple, l'émetteur 105 et le récepteur 110 sont positionnés en contact et à la surface de la peau d'un utilisateur, par exemple un animal ou un être humain, par exemple en vis-à-vis d'un vaisseau sanguin 118, par exemple une veine ou préférentiellement une artère. L'onde incidente 112 est par exemple au moins partiellement transmise par des tissus 116, par exemple comprenant une ou plusieurs couches de la peau, un ou plusieurs muscles, etc., avant d'atteindre le vaisseau sanguin 118. L'onde incidente 112 est par exemple au moins partiellement rétrodiffusée par les milieux rencontrés. L'onde rétrodiffusée 114 est détectée par le récepteur 110 du capteur optique 100. Une intensité de l'onde réfléchie 114 est inférieure à une intensité de l'onde incidente 112.

A titre de variante, une acquisition d'un signal par un capteur photopléthysmographique peut être effectuée en transmission, c'est-à-dire que l'émetteur 105 et le récepteur 110 se situent de part et d'autre de la zone à sonder, par exemple l'émetteur 105 est au-dessus de la zone à sonder et le récepteur 110 est en-dessous de la zone à sonder. Ce mode de réalisation est par exemple adapté à des régions relativement fines, par exemple un doigt ou un lobe d'une oreille, permettant à une partie du rayonnement de traverser la zone à sonder.

Dans l'exemple représenté, une onde de pouls 120, illustrée par une flèche sur la figure 1A et sur la figure 1B, circule dans le vaisseau sanguin 118.

Un diamètre du vaisseau sanguin 118 évolue au cours de la propagation de l'onde de pouls. La longueur d'onde de l'onde optique incidente 112 est par exemple choisie pour être absorbée plus fortement par le sang que par les tissus environnants. Lorsque le diamètre du vaisseau sanguin 118 augmente en vis-à-vis du capteur optique 100, le volume de sang à l'intérieur du vaisseau augmente localement, l'onde incidente 112 est par exemple relativement plus absorbée et l'intensité de l'onde rétrodiffusée 114, mesurée par le récepteur 110, diminue. Autrement dit, une augmentation du volume sanguin entraine une augmentation de l'absorption, et par conséquent une diminution de l'intensité de l'onde rétrodiffusée 114.

Le choix de la ou des longueurs d'onde utilisées influence par exemple l'amplitude d'une évolution du signal mesuré par le récepteur 110.

Le capteur optique 100 peut être utilisé quelle que soit l'orientation de l'artère par rapport aux positions de l'émetteur 105 et du récepteur 110.

L'émetteur 105 et le récepteur 110 sont par exemple séparés d'une distance L. La distance L est par exemple configurée pour discriminer des rayons rétrodiffusés ayant des trajectoires différentes et/ou pour discriminer une profondeur du vaisseau sanguin 118 sondé optiquement.

La figure 1C représente de façon schématique un exemple d'évolution d'un signal de photopléthysmographie (« PPG [a.u.] ») au cours du temps (« T[s] ») selon un mode de réalisation.

Le signal de photopléthysmographie est par exemple obtenu à l'aide du capteur optique 100 décrit en relation avec les figures 1A et 1B. Le signal de photopléthysmographie correspond par exemple à une intensité de l'onde rétrodiffusée 114. Une évolution du signal de photopléthysmographie est par exemple corrélée avec une évolution du volume sanguin dans la zone sondée, qui peut être reliée à une évolution du diamètre du vaisseau sanguin 118. Un tel signal est classiquement utilisé pour mesurer une fréquence cardiaque de l'utilisateur, mais ne permet pas directement de remonter à l'évolution au cours du temps d'une valeur du diamètre du vaisseau sanguin 118.

Selon un mode de réalisation de la présente description, un capteur ultrasonore est utilisé lors d'une phase de calibration pour établir une relation entre l'évolution du signal obtenu par le capteur optique 100 et l'évolution du diamètre du vaisseau sanguin 118. Lors de la phase de calibration, un modèle mathématique est défini, permettant de générer, à partir du seul signal optique, un signal représentatif de l'évolution au cours du temps d'une valeur représentative du diamètre du vaisseau sanguin. A l'issue de la phase de calibration, un signal représentatif de l'évolution au cours du temps d'une valeur représentative du diamètre du vaisseau sanguin est généré à partir des seules mesures fournies par le capteur optique.

La figure 2 représente de façon schématique, sous forme de blocs, un exemple d'un système 200 d'acquisition d'un signal représentatif d'un diamètre d'un vaisseau sanguin selon un mode de réalisation.

Le système 200 comprend un dispositif de traitement de données 210 (« CALC »), un capteur ultrasonore 220 (« US ») et un capteur optique 100 (« OPT »). Le capteur optique 100 correspond par exemple au capteur optique décrit en relation avec les figures 1A et 1B. Le capteur optique 100 comprend par exemple l'émetteur 105 (« EM ») et le détecteur 110 (« DET »). Le capteur ultrasonore 220 et le capteur optique 100 sont par exemple reliés au dispositif de traitement de données 210.

Le capteur ultrasonore 220 comprend un ou plusieurs transducteurs ultrasonores, par exemple une barrette linéaire ou une matrice de transducteurs ultrasonores.

Le capteur ultrasonore 220 est configuré pour générer une onde ultrasonore incidente dans les tissus 116 et pour détecter une onde ultrasonore réfléchie ou rétrodiffusée par les tissus 116 et le vaisseau sanguin 118, de manière à générer des signaux représentatifs de l'évolution de la structure du vaisseau sanguin 118. Ces signaux sont par exemple reconstruits sous la forme d'une ou plusieurs images, ou sont par exemple interprétés en termes d'échos pour localiser les différentes interfaces dans les tissus, et en particulier les parois de l'artère.

Le capteur ultrasonore 220 et le capteur optique 100 sont par exemple configurés pour effectuer des mesures du même vaisseau sanguin 118, par exemple à une même position ou à des positions relativement proches, par exemple distantes d'une distance inférieure ou égale à 1 cm, de préférence inférieure ou égale à 5 mm. A titre d'exemple, le capteur ultrasonore 220 et le capteur optique 100 sont configurés pour effectuer des mesures synchrones ou simultanées pendant la phase de calibration. Un recalage temporel est par exemple effectué par le dispositif de traitement de données 210 pour prendre en compte une distance entre les capteurs.

Le capteur ultrasonore 220 et le capteur optique 100 sont par exemple configurés pour transmettre les mesures au dispositif de traitement de données 210. Le dispositif de traitement de données 210 est par exemple configuré pour analyser les mesures. Le dispositif de traitement de données 210 comprend par exemple au moins un microprocesseur et au moins un circuit mémoire.

Selon un mode de réalisation, le dispositif de traitement de données 210 est configuré pour établir une relation entre une grandeur caractéristique du diamètre du vaisseau sanguin 118, obtenue à partir d'une mesure ultrasonore, et une mesure optique.

Selon un mode de réalisation, le dispositif de traitement de données 210 comprend deux unités de calcul : une première unité de calcul configurée pour effectuer une calibration du système 200 et une deuxième unité de calcul configurée pour utiliser la calibration sur une ou plusieurs mesures transmises par le capteur ultrasonore 220 et/ou le capteur optique 100. La première unité de calcul est par exemple configurée pour avoir une capacité de calcul relativement grande et la deuxième unité de calcul est par exemple configurée pour être moins encombrante et/ou pour consommer moins d'énergie que la première unité de calcul. A titre de variante, la calibration et les mesures sont effectuées par une même unité de calcul.

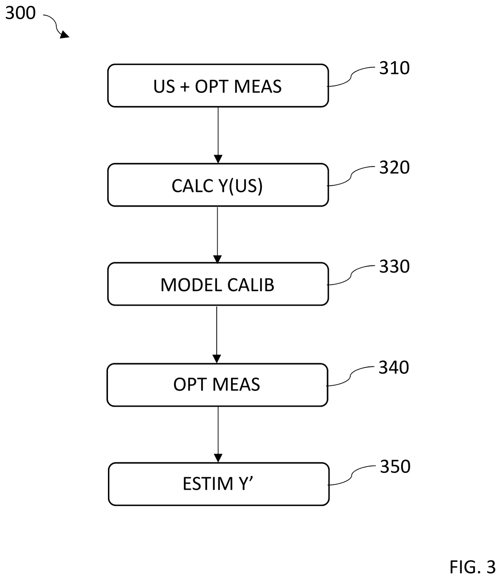

La figure 3 représente de façon schématique, sous forme de blocs, un exemple d'un procédé 300 d'acquisition d'un signal représentatif d'un diamètre d'un vaisseau sanguin selon un mode de réalisation. Le procédé 300 est par exemple mis en œuvre au moyen du système 200 de la figure 2.

Le procédé 300 comprend une étape 310 (« US + OPT MEAS ») de mesure de signaux optiques et ultrasonores représentatifs d'une évolution en fonction du temps du vaisseau sanguin 118 pendant une période de calibration.

Le capteur ultrasonore 220, décrit en relation avec la figure 2, est par exemple configuré pour, lors de l'étape 310, acquérir un ou plusieurs signaux ultrasonores d'une même région du vaisseau sanguin 118 pendant la période de calibration. Les signaux ultrasonores sont par exemple acquis successivement, par exemple avec une fréquence d'acquisition comprise entre 10 Hz et 1 kHz, et par exemple comprise entre 10 Hz et 500 Hz.

Le capteur optique 100, décrit en relation avec les figures 1A, 1B et 2, est par exemple configuré pour, lors de l'étape 310, effectuer une ou plusieurs mesures de signaux optiques représentatifs de l'évolution de la même région du vaisseau sanguin 118, pendant la même période de calibration. Le capteur ultrasonore 220 et le capteur optique 100 sont par exemple configurés pour effectuer des mesures synchrones.

A titre d'exemple, le capteur optique 100 est configuré pour effectuer une ou plusieurs mesures successives pour chacune d'une ou plusieurs configurations d'acquisition c. Une configuration d'acquisition c est par exemple définie par une longueur d'onde de l'onde incidente 112 et une distance L entre l'émetteur 105 et le détecteur 110 ou est par exemple définie par une longueur d'onde de l'onde incidente 112 et un couple comprenant un émetteur 105 et un détecteur 110 parmi une pluralité d'émetteurs et/ou de récepteurs. Chaque configuration d'acquisition c est par exemple appliquée simultanément ou successivement, dans un intervalle de temps court en comparaison à la fréquence d'acquisition.

Chaque couple comprenant un émetteur et un détecteur est par exemple positionné de façon pertinente pour sonder une zone d'intérêt.

Selon un mode de réalisation, plusieurs longueurs d'ondes sont utilisées par le capteur optique. Les tissus 116 comprennent par exemple différents éléments, par exemple un derme, un hypoderme, un ou plusieurs muscles, etc. Les différents éléments ont par exemple une composition et/ou des propriétés, en particulier des propriétés optiques, différentes. L'utilisation de plusieurs longueurs d'ondes permet par exemple de discriminer entre des variations de l'onde rétrodiffusée 114 causées par le vaisseau sanguin 118 et des variations causées par les tissus 116. Par exemple, une analyse spectrale est réalisée avec au moins deux longueurs d'ondes. En outre, l'absorption par les tissus 116 varie avec la longueur d'onde de l'onde incidente 112. La ou les longueurs d'ondes sont par exemple configurées pour sonder un volume, par exemple fixé, de tissus. Par exemple, une première longueur d'onde est utilisée pour une première acquisition pour sonder des tissus superficiels et une deuxième longueur d'onde est utilisée pour une deuxième acquisition pour sonder les tissus superficiels et profonds ainsi que le ou les vaisseaux sanguins 118. Une opération est par exemple effectuée entre le signal de la première acquisition et le signal de la deuxième acquisition pour s'abstraire des perturbations induites par les tissus superficiels. Par exemple, le signal de la première acquisition est soustrait au signal de la deuxième acquisition. Le capteur optique 100 comprend par exemple plusieurs émetteurs 105 et/ou plusieurs récepteurs 110. A titre d'exemple, chaque émetteur 105, par exemple une diode, est configuré ou choisi pour émettre à une longueur d'onde spécifique ou fixée et chaque récepteur 110, par exemple une photodiode, est configuré pour détecter une plage de longueurs d'onde ou une onde ayant une longueur d'onde fixée, par exemple au moyen d'un ou plusieurs filtres. Les émetteurs 105 sont par exemple relativement proches les uns des autres pour sonder le même vaisseau sanguin 118.

Selon un autre mode de réalisation, plusieurs distances L sont utilisées, par exemple pour une longueur d'onde ou pour plusieurs longueurs d'ondes. Un volume de tissus fixé est par exemple associé avec la distance L. A titre d'exemple, le récepteur 110 comprend plusieurs éléments de détection, par exemple plusieurs photodiodes, chaque élément de détection ayant une distance fixée avec l'émetteur 105.

Selon un mode de réalisation, au moins deux configurations d'acquisition c sont effectuées, par exemple pour deux longueurs d'ondes et une distance ou pour deux distances et une longueur d'onde. A titre d'exemple, des acquisitions sont effectuées pour plusieurs longueurs d'ondes et/ou plusieurs distances, de manière séquentielle ou simultanée. Un grand nombre de configurations d'acquisition a l'avantage d'augmenter un contenu informationnel collecté pour mieux extraire un signal d'intérêt.

Par exemple, le système 200 est configuré pour que le capteur optique 100 enregistre des signaux dans m configurations d'acquisition, notées c1, ..., cm, pendant la période de calibration, où m correspond à :
[Math 1] $m = {\sum }_{p = 1}^{k} {L}_{p}$
où k désigne le nombre de longueurs d'ondes distinctes pour lesquelles des signaux sont enregistrés, avec k entier supérieur ou égal à 1, de préférence supérieur ou égal à 2, et p est un entier allant de 1 à k. Lₚ désigne le nombre de distances L distinctes entre les émetteurs 105 et leurs détecteurs 110 pour la longueur d'onde de rang p, avec Lₚ entiers supérieurs ou égaux à 1, de préférence supérieurs ou égaux à 2. Le système 200 est par exemple en outre configuré pour que le capteur optique 100 enregistre des signaux pour n pas ou intervalles de temps, notés t1, ..., tn, pendant la période de calibration, avec n entier supérieur ou égal à 1, par exemple strictement supérieur à 1, par exemple supérieur ou égal à 10, par exemple supérieur ou égal à 100. Pour chaque pas de temps, le capteur optique 100 effectue par exemple m mesures, par exemple correspondant à une mesure par configuration d'acquisitions. Les n pas de temps sont par exemple régulièrement répartis tout au long de la période de calibration, ou sont par exemple irrégulièrement répartis tout au long de la période de calibration. A titre d'exemple, non limitatif, le capteur ultrasonore 220 est configuré pour acquérir des signaux ultrasonores aux mêmes n pas de temps que le capteur optique tout au long de la phase de calibration.

Le capteur ultrasonore 220 et le capteur optique 100 sont par exemple configurés pour transmettre les signaux mesurés pendant ou à l'issue de la période de calibration au dispositif de traitement de données 210.

Le procédé 300 comprend en outre, après l'étape 310, une étape 320 (« CALC Y(US) ») de détermination d'un signal Y représentatif de l'évolution d'un diamètre du vaisseau sanguin 118 au cours de la période de calibration, à partir des signaux acquis par le capteur ultrasonore 220 pendant la période de calibration.

A titre d'exemple, le dispositif de traitement de données 210 détermine un vecteur de calibration *Ỹₙ* défini par : ${{Y}^{˜}}_{n} = \left(\begin{matrix}y 1 \\ ⋮ \\ yn\end{matrix}\right)$, où y1, ..., yn correspondent respectivement à des valeurs représentatives d'une grandeur caractéristique du diamètre du vaisseau sanguin 118 déterminées à partir des mesures ultrasonores pour chacun des n pas de temps de la période de calibration. En particulier, on note yj ou y(tj) la valeur représentative d'une grandeur caractéristique du diamètre du vaisseau sanguin 118 déterminée pour le pas de temps tj, avec j entier allant de 1 à n.

Par convention, dans cette description, les valeurs nommées avec un tilde représentent des données de calibration.

A titre d'exemple, le vaisseau sanguin 118 n'est pas cylindrique et la grandeur caractéristique du diamètre du vaisseau sanguin 118 correspond à l'aire d'une section du vaisseau sanguin 118 ou à un diamètre moyen de la section du vaisseau sanguin 118 ou à une hauteur du vaisseau sanguin 118 dans un plan vertical ou au diamètre d'un cercle ayant la même surface que celle de la section du vaisseau considéré, ou à toute autre dimension caractéristique de la section du vaisseau sanguin. Par exemple, la grandeur caractéristique Y du diamètre du vaisseau sanguin 118 correspond au diamètre d'un cercle ayant l'aire A de la section du vaisseau sanguin 118 de sorte que : $Y \left(t\right) = 2 \sqrt{\frac{A \left(t\right)}{π}}$ avec t une variable temporelle.

La détermination d'une grandeur caractéristique du diamètre d'un vaisseau sanguin à partir de mesures effectuées au moyen d'un capteur ultrasonore est connue de la personne du métier et ne sera pas détaillée davantage.

Le procédé 300 comprend en outre, après les étapes 310 et 320, une étape 330 (« MODEL CALIB ») de détermination, par le dispositif de traitement de données 210, d'un modèle mathématique permettant de générer, à partir du seul signal optique, un signal Y représentatif de l'évolution au cours du temps d'une valeur représentative du diamètre du vaisseau sanguin. A titre d'exemple, l'étape 330 comprend le calcul d'un vecteur de paramètre p comprenant au moins deux coefficients de calibration du modèle par le dispositif de traitement de données 210.

Les coefficients de calibration sont calculés à partir des n grandeurs caractéristiques du diamètre du vaisseau sanguin 118 déterminées à l'étape 320 à partir des mesures ultrasonores, et à partir des n*m valeurs de signaux optiques mesurées à l'étape 310.

Les n*m valeurs de signaux optiques mesurées à l'étape 310 sont rassemblées dans une matrice de calibration *S̃ₙₓₘ* définie par :
${{S}^{˜}}_{nxm} = \left(\begin{matrix}{s}^{˜} 11 & ⋯ & {s}^{˜} 1 m \\ ⋮ & ⋱ & ⋮ \\ {s}^{˜} n 1 & ⋯ & {s}^{˜} nm\end{matrix}\right)$
où *s̃*ij désigne un signal mesuré au moyen du capteur optique 100 pour la configuration d'acquisition cj et au pas de temps ti, avec j entier allant de 1 à m.

Selon un mode de réalisation le modèle mathématique est défini à l'aide d'une fonction f de sorte que :
[Math 2] $Y \left(t\right) = f \left(\left\{{S}_{c}\left(t\right)\right\}, p\right)$

où t est une variable temporelle, S_{c} correspond au signal mesuré par le capteur optique 220 pour la configuration d'acquisition c, c'est-à-dire que S_{c} correspond au vecteur $\left(\begin{matrix}sc 1 \\ ⋮ \\ scn\end{matrix}\right) = \left(\begin{matrix}sc \left(t1\right) \\ ⋮ \\ sc \left(tn\right)\end{matrix}\right)$, et p désigne le vecteur de paramètre.

Selon un premier mode de réalisation, un modèle linéaire est utilisé et une régression linéaire est effectuée par le dispositif de traitement de données 210 pour déterminer les coefficients de calibration.

Le modèle linéaire est défini par :
[Math 3] $Y = p 0 + {S}_{nxm} ∗ {P}_{m}$
où :
- Sₙₓₘ est une matrice comprenant des valeurs de signaux optiques ;
- p0 est un coefficient de calibration ; et
- Pₘ correspond au vecteur $\left(\begin{matrix}p 1 \\ ⋮ \\ pm\end{matrix}\right)$ où p1, ..., pm sont des coefficients de calibration.

On note par exemple $p = \left(\begin{matrix}p 0 \\ {P}_{m}\end{matrix}\right)$.

Effectuer une régression linéaire pour résoudre l'équation (2) revient par exemple à résoudre le système suivant :
[Math 4] ${argmin}_{p} \left‖p0+{{S}^{˜}}_{nxm}∗{P}_{m}-{{Y}^{˜}}_{n}\right‖$
où :
- argmin correspond à l'argument minimum d'une fonction, c'est-à-dire à la valeur du vecteur de paramètre p pour laquelle la fonction atteint son minimum ; et
- ∥.∥ correspond à une norme d'un vecteur, par exemple la norme euclidienne.

Une méthode des moindres carrés est par exemple utilisée pour résoudre le système de l'équation (4) et pour déterminer les coefficients de calibration p0, p1, ..., pm.

Dans ce mode de réalisation, la somme du nombre de pas de temps d'acquisition n et du nombre de configurations d'acquisition m est supérieur ou égal à trois. Par exemple, n est supérieur ou égal à 1 et m est supérieur ou égal à 2. A titre de variante, n est supérieur ou égal à 2 et m est supérieur ou égal à 1.

Selon un deuxième mode de réalisation, une régression linéaire régularisée, par exemple en utilisant la méthode Lasso, est effectuée par le dispositif de traitement de données 210 pour déterminer les coefficients de calibration. Le modèle est également défini par l'équation (3).

Les coefficients de calibration sont par exemple déterminés en résolvant le système suivant utilisant la fonction de coût de Lasso :
[Math 5] ${argmin}_{p} \left[\frac{1}{2 n}\left‖p0+{{S}^{˜}}_{nxm}∗{P}_{m}-{{Y}^{˜}}_{n}\right‖+λ{\sum }_{j = 1}^{m} \left|{p}_{j}\right|\right]$
où :
- λ est un paramètre de régularisation ; et
- ∥.∥ correspond à la norme d'un vecteur, par exemple la norme euclidienne.

Une méthode d'optimisation non-linéaire est par exemple utilisée pour résoudre l'équation (5) et pour déterminer les coefficients de calibration p0, p1, ..., pm.

Dans ce mode de réalisation, la somme du nombre de pas de temps d'acquisition n et du nombre de configurations d'acquisition m est supérieur ou égal à trois.

Plus le paramètre de régularisation λ est grand, plus le terme $λ {\sum }_{j = 1}^{m} \left|{p}_{i}\right|$ de l'équation (5) est grand, et plus la résolution aura tendance à rendre nuls certains coefficients de calibration. A titre d'exemple, lors d'une première phase de calibration, la résolution de l'équation (5) est réalisée pour plusieurs valeurs de A. Ainsi, il est par exemple possible de déterminer un nombre de configurations d'acquisition m à effectuer et combiner pour préserver la qualité de la phase de calibration et réduire le nombre de configurations d'acquisition à effectuer lors de phases de calibrations ultérieures. Un avantage de cette méthode est une réduction de la durée de la phase de calibration et une réduction du temps de calcul associé avec la détermination d'un nombre de configurations d'acquisition m plus faible.

Selon un troisième mode de réalisation, un modèle physique, par exemple se basant sur une approximation de l'équation de Beer-Lambert, est utilisée pour modéliser une variation de l'intensité du signal optique en sortie des tissus 116. A titre d'exemple, les tissus 116 sont considérés homogènes avec un coefficient d'atténuation effectif µ_{eff} constant. L'équation de l'intensité du signal optique *S_{cj}* mesuré par le capteur optique 100 est donné par :
[Math 6] ${S}_{cj} \left(t\right) = {S}_{cj}^{0} {e}^{- {μ}_{eff} . {L}_{t}} {e}^{- {μ}_{art} . {K}_{j} {Y}_{j} \left(t\right)}$
où :
- ${S}_{cj}^{0}$correspond à l'intensité lumineuse de l'onde incidente 112 en sortie de l'émetteur 105 du capteur optique 100, dans la configuration d'acquisition cj ;
- µ_{eff} correspond au coefficient d'atténuation effectif prenant en compte la perte d'énergie due à l'absorption et à la diffusion de la lumière dans la partie considérée comme statique du milieu ;
- Lt correspond à une longueur d'interaction caractéristique parcourus par la lumière dans les tissus 116 ;
- Yⱼ correspond au signal Y, déterminé, dans ce mode de réalisation, à partir des mesures de signaux optiques acquis lors de l'étape 310 pour la configuration d'acquisition cj ;
- µₐᵣₜ correspond au coefficient d'atténuation effectif prenant en compte la diffusion et l'absorption dans le vaisseau sanguin 118 ; et
- Kⱼ est un coefficient fixé et donné pour une configuration cj reliant une longueur d'interaction caractéristique de la lumière dans le vaisseau sanguin 118 à la grandeur caractéristique du diamètre du vaisseau sanguin 118 Yⱼ.

Le produit Kⱼ.Yⱼ correspond par exemple à une longueur d'interaction caractéristique de lumière dans le vaisseau sanguin 118.

L'équation (6) peut se mettre sous la forme de :
[Math 7] ${Y}_{j} \left(t\right) = bj + aj . ln \left(\frac{{S}_{cj} \left(t\right)}{{S}_{cj}^{0}}\right)$
avec $bj = - \frac{1}{{μ}_{art} . {K}_{j}}$ et $aj = - \frac{{μ}_{eff} . {L}_{t}}{{μ}_{art} . {K}_{j}}$. On note ${D}_{cj} = ln \left(\frac{{S}_{cj} \left(t\right)}{{S}_{cj}^{0}}\right)$ et ${{D}^{˜}}_{cj} = ln \left(\frac{{{S}^{˜}}_{cj}}{{S}_{cj}^{0}}\right)$ avec ${{S}^{˜}}_{cj} = \left(\begin{matrix}si 1 \\ ⋮ \\ sin\end{matrix}\right) = \left(\begin{matrix}{S}_{cj} \left(t1\right) \\ ⋮ \\ {S}_{cj} \left(tn\right)\end{matrix}\right)$.

Lt correspond à une longueur d'interaction caractéristique de l'onde optique avec les tissus 116

On note par exemple $p = \left(\begin{matrix}aj \\ bj\end{matrix}\right)$.

A titre d'exemple, les coefficients aj et bj sont par exemple déterminés à l'aide de deux couples de valeurs {*S*_{*c*1}(t1), y(t1)} et {(*S*_{*c*1}(t2), y(t2)}.

Un nombre de mesures supérieur à deux est par exemple effectué pour une même configuration c1 d'acquisition et le couple de valeurs {*S*_{*c*1}(t1), y(t1)} correspond au couple dont l'intensité *S*_{*c*1}(t1) est maximale et le couple de valeurs *{S*_{c1}(t2), y(t2)} correspond au couple dont l'intensité *S*_{*c*1}(t2) est minimale.

La constante aj et la constante bj sont par exemple déterminées par une résolution d'une équation linéaire à deux inconnues, pour laquelle au moins deux mesures sont utilisées, ou par exemple en utilisant une méthode de moindre-carré.

La constante aj est égale à :
[Math 8] $aj = \frac{y 1 - y 2}{ln \left(\frac{s 1}{s 2}\right)}$
avec y1=y(t1), y2=y(t2), s1=*S*_{*c*1}(t1) et s2=*S*_{*c*1}(t2).

La constante bj est égale à :
[Math 9] $bj = {y}_{1} - \frac{{y}_{1} - {y}_{2}}{ln \left(\frac{{s}_{1}}{{s}_{2}}\right)} ln \left(\frac{{s}_{1}}{{s}_{0}}\right)$
avec y1=y(t1), y2=y(t2), s1=*S*_{*c*1}(t1 et s2=*S*_{*c*1}(t2).

Selon un autre exemple, lorsque n est différent de deux, les coefficients de calibration sont déterminés par la résolution de :
[Math 10] ${argmin}_{p} \left‖aj∗{{D}^{˜}}_{cj}+bj-{{Y}^{˜}}_{n}\right‖$

Une méthode des moindres carrés est par exemple utilisée pour résoudre le système de l'équation (10) et pour déterminer les coefficients de calibration aj et bj.

Dans ce mode de réalisation, le nombre de pas de temps d'acquisition n est supérieur à deux et le nombre de configurations d'acquisition m est supérieur ou égal à un.

Selon un quatrième mode de réalisation, la méthode précédente est répétée pour chaque configuration d'acquisition ci.

A titre d'exemple, le signal Y correspond à la moyenne des signaux Yᵢ déterminés avec la méthode précédente pour chaque configuration d'acquisition ci.

On note Dₙₓₘ une matrice obtenue en concaténant les vecteurs D_{ci} et on note *D̃*ₙₓₘ une matrice obtenue en concaténant les vecteurs *D̃*_{cj}. On note ${A}_{m} = \left(\begin{matrix}a 1 \\ ⋮ \\ am\end{matrix}\right)$ et ${B}_{m} = \left(\begin{matrix}b 1 \\ ⋮ \\ bm\end{matrix}\right)$ .

Le modèle est ainsi défini par :
[Math 11] $Y = \frac{1}{m} {D}_{nxm} ∗ {A}_{m} + \frac{1}{m} {1}_{nxm} ∗ {B}_{m}$
où 1ₙₓₘ correspond à la matrice identité de taille nxm.

On note $p = \left(\begin{matrix}{A}_{m} \\ {B}_{m}\end{matrix}\right)$.

Les coefficients de calibration sont déterminés par la résolution de :
[Math 12] ${argmin}_{p} \left‖\frac{1}{m}\left({{D}^{˜}}_{nxm}{1}_{nxm}\right)∗p-{{Y}^{˜}}_{n}\right‖$

Une méthode des moindres carrés, par exemple complétée par une régularisation des paramètres, est par exemple utilisée pour résoudre le système de l'équation (12) et pour déterminer le vecteur de paramètre p.

Selon un cinquième mode de réalisation, un modèle physique M relativement complexe est considéré et une simulation numérique est utilisée pour déterminer les coefficients de calibration et résoudre l'équation (2) à partir des mesures optiques effectuées à l'étape 310 et des valeurs de la grandeur caractéristique du diamètre du vaisseau sanguin 118 déterminées à l'étape 320 du procédé 300.

Le modèle physique M s'exprime par exemple sous une forme littérale, par une approche numérique, par exemple à l'aide d'éléments finis, ou par une simulation de type Monte-Carlo.

Le modèle, pour une configuration d'acquisition cj, est par exemple défini par :
[Math 13] ${S}_{cj} \left(t\right) = M \left(Y\left(t\right),p\right)$

Le vecteur de paramètre p rassemble par exemple des paramètres du modèle qui devront être adaptés au sujet, par exemple des coefficients d'absorption et des grandeurs physionomiques, par exemple un diamètre nominal, une profondeur du vaisseau sanguin 118, etc.

Les coefficients de calibration sont déterminés par la résolution de :
[Math 14] ${argmin}_{p} \left‖M\left({{Y}^{˜}}_{n}, p\right)-{{S}^{˜}}_{nxm}\right‖$

Le signal Y est ensuite déterminé en résolvant :
[Math 15] ${argmin}_{Y} \left‖M\left(Y, p\right)-{S}_{nxm}\right‖$

Selon un sixième mode de réalisation, une méthode d'apprentissage supervisé est utilisée.

Lors d'une phase d'apprentissage d'un algorithme, les données synchrones {*Ỹₙ,S̃ₙₓₘ*} sont utilisées pour l'entraînement d'un modèle R. Le signal Y est ensuite déterminé selon :
[Math 16] $Y = R \left({S}_{nxm}, p\right)$

Les étapes 310, 320 et 330 du procédé 300 correspondent par exemple à une phase de calibration du procédé 300.

Le procédé 300 comprend par exemple en outre, après la phase de calibration, une étape 340 (« OPT MEAS ») de mesure optique par le capteur optique 100 du système 200 pendant une période d'acquisition.

Le capteur optique 100 est par exemple configuré pour mesurer des signaux optiques S*ₙₓₘ'* représentatifs d'une évolution du vaisseau sanguin 118 pendant la période d'acquisition.

Le procédé 300 comprend par exemple en outre, après l'étape 340, une étape 350 (« ESTIM Y' ») de détermination d'un signal Y' représentatif de l'évolution d'une grandeur caractéristique du diamètre du vaisseau sanguin 118 par le dispositif de traitement de données 210 du système 200.

Le dispositif de traitement de données 210 utilise le modèle mathématique déterminé à l'étape 330 et défini par l'équation (2), ou par l'une des équations (3), (7), (11), (15) et (16), pour calculer le signal Y' à partir des mesures optiques S*ₙₓₘ'* effectuées à l'étape 340.

Un avantage de ce procédé est que la phase de calibration comprenant les étapes 310, 320 et 330 est par exemple effectuée une unique fois ou est par exemple effectuée périodiquement, par exemple à une fréquence comprise entre une fois toutes les 20 minutes et une fois par semaine. Entre deux phases de calibration, le dispositif de traitement de données 210 est par exemple configuré pour déterminer une grandeur représentative du diamètre du vaisseau sanguin 118 à partir des seules mesures effectuées par le capteur optique 100, selon les étapes 340 et 350 du procédé 300, sans utiliser le capteur ultrasonore. Ceci permet, lors des phases d'acquisition entre deux phases de calibration successives, de bénéficier de mesures fiables, avec une consommation énergétique limitée et des temps de calcul réduits, et une réduction des performances de calcul requises.

A titre d'exemple, le capteur optique 100 est intégré dans un accessoire portable, par exemple un bracelet, une montre, un patch, un brassard, etc. Le dispositif de traitement de données 210 est par exemple intégré à l'accessoire portable ou est par exemple dans un dispositif externe, par exemple un ordinateur, un téléphone portable, etc. Par exemple, les mesures effectuées par le capteur optique 100 sont envoyées, par une liaison filaire ou non-filaire, par exemple une liaison radio, au dispositif de traitement de données 210. Les mesures sont par exemple envoyées après chaque acquisition ou périodiquement ou par un transfert de données, manuel ou automatique, effectué par l'utilisateur.

Selon un mode de réalisation, le capteur ultrasonore 220 est intégré à l'accessoire. Un avantage du procédé 300 est alors une réduction de la consommation de l'accessoire et une réduction du temps de traitement des données résultant du fait que le capteur ultrasonore 220 est utilisé uniquement pendant les phases de calibration.

Selon un autre mode de réalisation, le capteur ultrasonore 220 est un dispositif externe à l'accessoire, par exemple un échographe relativement volumineux, par exemple non portatif, couplé à l'accessoire uniquement lors des phases de calibration. Un avantage supplémentaire du procédé 300 est alors une réduction de la taille de l'accessoire, par exemple entrainant un inconfort moindre pour l'utilisateur et/ou facilitant une acquisition tout au long de la journée et améliorant la compatibilité de l'accessoire avec des activités de l'utilisateur. Un autre avantage de mesures effectuées au moyen d'un capteur optique est que l'utilisation d'un gel n'est pas nécessaire pour obtenir un signal qualitatif.

Un avantage des divers modes de réalisation présentés est l'obtention d'une grandeur caractéristique du diamètre d'un vaisseau sanguin, par exemple d'une artère, et de son évolution au cours du temps, à partir de mesures acquises par un capteur optique. Les dispositifs détaillés permettent en outre une mesure d'une grandeur caractéristique du diamètre d'un vaisseau sanguin de manière continue au cours des activités quotidiennes, ce qui est par exemple difficile avec un brassard et impossible avec un échographe. Une valeur quantitative, dans une unité de longueur, de la grandeur caractéristique du diamètre est obtenue et peut être directement comparée à des mesures obtenues au moyen d'un capteur ultrasonore. Un autre avantage des divers modes de réalisation présentés est l'obtention de valeurs fiables et un procédé adapté à la diversité des utilisateurs et des configurations d'acquisition.

Une application des dispositifs détaillés dans la présente description et du procédé 300 est un suivi médical, par exemple sur une période de temps étendue. Une autre application est un suivi de l'activité cardiaque, par exemple pour un sportif.

La mesure d'une évolution du diamètre d'un vaisseau sanguin, d'une artère par exemple, au cours du temps permet un suivi de paramètres cardio-vasculaires tels que la pression artérielle, le rythme cardiaque, la saturation pulsée de l'hémoglobine en oxygène (SpO2), la rigidité artérielle, le pouls, etc.

Divers modes de réalisation et variantes ont été décrits. La personne du métier comprendra que certaines caractéristiques de ces divers modes de réalisation et variantes pourraient être combinées, et d'autres variantes apparaîtront à la personne du métier. En particulier, le récepteur 110 du capteur optique 100 est par exemple un récepteur matriciel. Les étapes de calibration 210, 220 et 100 du procédé 300 sont par exemple effectuées pour différentes unités du récepteur afin de déterminer les unités permettant une calibration optimale. Une qualité de la calibration est par exemple déterminée en mesurant un écart quadratique moyen entre les mesures optiques et les mesures ultrasonores correspondantes effectuées à l'étape 310.

Selon un mode de réalisation, la phase de calibration est réalisée plusieurs fois en utilisant des méthodes de résolution distinctes pour déterminer la méthode de résolution résultant en la calibration de meilleure qualité. Cette méthode de résolution est par exemple sélectionnée pour de futures phases de calibration.

Enfin, la mise en œuvre pratique des modes de réalisation et variantes décrits est à la portée de la personne du métier à partir des indications fonctionnelles données ci-dessus.

## Revendications

1. Procédé d'acquisition d'un signal représentatif d'un diamètre d'un vaisseau sanguin (118), comprenant une phase de calibration, la phase de calibration comprenant les étapes suivantes :
1) déterminer un signal de référence (*Ỹₙ*) représentatif de l'évolution du diamètre du vaisseau sanguin pendant une période de calibration, au moyen d'un capteur ultrasonore (220) ;
2) acquérir un signal optique (*S̃ₙₓₘ*) représentatif de l'évolution du vaisseau sanguin pendant la période de calibration, au moyen d'un capteur photopléthysmographique (100) ;
3) déterminer, à partir du signal optique mesuré à l'étape 2) et du signal de référence déterminé à l'étape 1), un modèle mathématique adapté à générer, à partir du seul signal optique mesuré à l'étape 2), un signal estimé (Y) représentatif de l'évolution du diamètre du vaisseau sanguin pendant la période de calibration,
le procédé comprenant en outre, après la phase de calibration, les étapes suivantes :
4) mesurer un signal optique représentatif de l'évolution du vaisseau sanguin pendant une période d'acquisition, au moyen du capteur photopléthysmographique (100) ; et
5) calculer un signal estimé (Y') représentatif de l'évolution du diamètre du vaisseau sanguin pendant la période d'acquisition, à partir du signal mesuré à l'étape 4) et du modèle mathématique déterminé à l'étape 3).

2. Procédé d'acquisition selon la revendication 1, dans lequel, l'étape 1) comprend l'acquisition d'un ou plusieurs signaux ultrasonores et la détermination du signal de référence (***Ỹₙ***) à partir desdits signaux ultrasonores.

3. Procédé d'acquisition selon la revendication 1 ou 2, dans lequel le signal optique acquis à l'étape 2) comprend une pluralité de signaux optiques acquis respectivement dans plusieurs configurations d'acquisitions (c).

4. Procédé d'acquisition selon la revendication 3, dans lequel le capteur photopléthysmographique (100) comprend au moins un émetteur (105) et au moins un récepteur (110) et dans lequel une configuration d'acquisition (c) comprend une longueur d'onde d'émission par l'émetteur (105) et une distance entre l'émetteur (105) et le récepteur (110).

5. Procédé d'acquisition selon l'une quelconque des revendications 1 à 4, dans lequel, à l'étape 3), le modèle mathématique est de la forme : $Y \left(t\right) = f \left(\left\{S\left(t\right)\right\}, p\right)$ où Y désigne le signal estimé, t désigne une variable temporelle, f désigne une fonction déterminée, S désigne le signal optique acquis à l'étape 2) et p désigne un vecteur de paramètre comprenant un ou plusieurs paramètres à déterminer.

6. Procédé d'acquisition selon la revendication 5, dans lequel la fonction f est une fonction linéaire, le vecteur de paramètre p de la fonction linéaire étant déterminé par régression linéaire.

7. Procédé d'acquisition selon la revendication 6, dans lequel ladite régression linéaire est une régression linéaire régularisée, par exemple une régression Lasso.

8. Procédé d'acquisition selon la revendication 5, dans lequel la fonction f est définie comme suit : $f \left(\left\{S\left(t\right)\right\}, p\right) = A ∗ ln \left(S\left(t\right)/{S}_{0}\right) + B$ où S₀ désigne l'intensité d'une onde émise (112) par le capteur photopléthysmographique (100) à l'étape 2) et A et B sont deux valeurs de paramètres du vecteur de paramètre p.

9. Procédé d'acquisition selon la revendication 8, dans lequel : $A = \frac{\left(y1-y2\right)}{ln \left(\frac{s 1}{s 2}\right)}$ et $B = {y}_{1} - \frac{{y}_{1} - {y}_{2}}{l n \left(\frac{{s}_{1}}{{s}_{2}}\right)} ln \left(\frac{{s}_{1}}{{s}_{0}}\right)$ où y1 et y2 désignent deux valeurs de la grandeur représentative du diamètre du vaisseau sanguin déterminée à l'étape 3) et s1 et s2 désignent deux valeurs du signal optique mesuré à l'étape 2), y1 étant obtenue à partir d'une première mesure synchrone avec s1 et y2 étant obtenue à partir d'une deuxième mesure synchrone avec s2.

10. Procédé d'acquisition selon la revendication 5, dans lequel la fonction f est définie par simulation numérique.

11. Procédé d'acquisition selon la revendication 5, dans lequel un apprentissage supervisé est utilisé pour déterminer la fonction f.

12. Procédé d'acquisition selon l'une quelconque des revendications 1 à 11, dans lequel le vaisseau sanguin (118) est une artère.

13. Système d'acquisition d'un signal représentatif d'un diamètre d'un vaisseau sanguin (118) comprenant :
- un capteur ultrasonore (220) ;
- un capteur photopléthysmographique (100) ; et
- un dispositif de traitement de données (210) configuré pour, lors d'une phase de calibration :
1) déterminer un signal de référence représentatif de l'évolution du diamètre du vaisseau sanguin pendant une période de calibration, au moyen du capteur ultrasonore (220) ;
2) acquérir un signal optique (*S̃ₙₓₘ*) représentatif de l'évolution du vaisseau sanguin pendant la période de calibration, au moyen du capteur photopléthysmographique (100) ;
3) déterminer, à partir du signal optique mesuré à l'étape 2) et du signal de référence déterminé à l'étape 1), un modèle mathématique adapté à générer, à partir du seul signal optique mesuré à l'étape 2), un signal estimé (Y) représentatif de l'évolution du diamètre du vaisseau sanguin pendant la période de calibration,
le dispositif de traitement de données (210) étant en outre configuré pour exécuter, après la phase de calibration, les étapes suivantes :
4) mesurer un signal optique représentatif de l'évolution du vaisseau sanguin pendant une période d'acquisition, au moyen du capteur photopléthysmographique (100) ; et
5) calculer un signal estimé (Y') représentatif de l'évolution du diamètre du vaisseau sanguin pendant la période d'acquisition, à partir du signal mesuré à l'étape 4) et du modèle mathématique déterminé à l'étape 3).
